# EUROPEAN PATENT SPECIFICATION

(11) **EP 0 327 000 B1**
(45) Date of publication and mention of the grant of the patent: **28.07.1993**
(21) Application number: 89101583.6
(22) Date of filing: 30.01.1989
(51) Int. Cl.: C12N 15/13, A61K 39/395, C12N 5/10, C12P 21/08

(54) **Gene fragments coding for the variable region of an anti-HIV antibody, chimeric anti-HIV antibodies expressed using the same, and process for their preparation**
Genfragmente, die die variable Region eines Anti-HIV-Antikörpers codieren, von diesen exprimierte chimäre Anti-HIV-Antikörper und Verfahren zu deren Herstellung
Fragments de gènes codant pour la région variable d'un anticorps anti-HIV, anticorps chimériques anti-HIV exprimés lors de leur utilisation et procédé pour leur préparation

(30) Priority: 30.01.1988 JP 20255/88; 08.07.1988 JP 171385/88
(43) Date of publication of application: 09.08.1989
(73) Proprietor: Juridical Foundation The Chemo-Sero-Therapeutic Research Institute, Kumamoto-shi Kumamoto-ken (JP)
(72) Inventor: Maeda, Hiroaki, Kumamoto-shi Kumamoto-ken (JP); Eda, Yasuyuki, Kikuchi-gun Kumamoto-ken (JP); Kimachi, Kazuhiko, Kumamoto-shi Kumamoto-ken (JP); Tokiyoshi, Sachio, Kumamoto-shi Kumamoto-ken (JP); Matsushita, Shuzo, Kumamoto-shi Kumamoto-ken (JP); Hattori, Toshio, Shin-Ooe, Kumamoto-shi Kumamoto-ken (JP); Takatsuki, Kiyoshi, Kumamoto-shi Kumamoto-ken (JP)
(74) Representative: VOSSIUS & PARTNER

(56) References cited:
- WO-A-88/09181
- CANCER RESEARCH, vol. 47, 15th February 1987, pages 999-1005; Y. NISHIMURA et al.: "Recombinant human-mouse chimeric monoclonal antibody specific for common acute lymphocytic leukemia antigen"
- EUROPEAN JOURNAL OF IMMUNOLOGY, vol. 16, 1986, pages 1465-1468; T.C. CHANH et al.: "Human immunodeficiency virus gp 120 glycoprotein detected by a monoclonal antibody to a synthetic peptide"
- Archives of Aids Research 1, 333 - 341 (1987)
- Journal of Virology 64, 3674 - 3678 ( 1990)
- Nature 355, 728 - 730 (1992)

## Description

The present invention relates to novel chimeric anti-human immunodeficiency virus (HIV) antibodies which are useful for the treatment and prevention of the acquired immunodeficiency syndrome (AIDS) caused by HIV. More particularly, the present invention relates to gene fragments coding for variable regions of the H and L chains of the chimeric anti-HIV antibodies which are capable of expressing chimeric anti-HIV antibodies having an HIV-neutralizing activity, to a transformant into which such gene fragments are incorporated, to chimeric anti-HIV antibodies expressed therefrom and to a process for preparing the same.

AIDS is a viral disease caused by the retrovirus HIV [Barre-Sinoussi et al., Science, 220, 868 (1983); Popovic et al., Science, 224, 497 (1984)]. This disease was discovered in five homosexuals in Los Angeles that suffered from Kalini pneumonia in 1981 as reported by the American CDC Weekly [Centers for Disease Control: MMWR, 30, 250 (1981)]. Since then, this disease has very rapidly spread all over the world. According to a statistical report by the World Health Organization (WHO), this disease has already been found in 122 countries and the total number of patients exceeded 60,000 as of August 12, 1987. Also in Japan, fifty patients have been confirmed as of September 4, 1987, twenty eight patients thereof having already died. Although AIDS has spread all over the world so rapidly, prevention and treatment of this disease are not yet effectively possible.

One anti-HIV agent put to practical use is AZT (3′-azido-3′-deoxythymidine) [Carroll Ezzell, Nature, 326, 430 (1987)]. This drug was developed as a carcinostatic agent and acts early in the viral life cycle by blocking the reverse transcriptase (RT) and thus the reverse transcription of the RNA genome of HIV-1 into DNA. However, it has been found that this drug shows a strong toxicity against active hematopoietic tissue, causing anemia in many cases. Many other substances have been studied for anti-HIV activity, but an effective and safe anti-HIV agent has not yet been developed. Although an intensive study has been continued to develop a vaccine for the prevention of this disease, hitherto there has not yet been reported any vaccine suitable for practical use.

A clue for immunotherapy may have been found in comparison of the immunological profiles of serologically positive healthy individuals with those of patients with AIDS-related complex (ARC) and AIDS. Neutralizing antibodies against HIV were frequently detected in infected individuals, and low or absent neutralizing titers correlated with poor prognosis. The level of antibody relates with the presence of antibody dependent cell cytotoxicity (ADCC) or inhibits the function of RT which is present in the asymptomatic phase but declines in the symptomatic phase [Ho et al., J. Virology, 61, 2024 (1987)]. Further a relation between clinics and neutralizing antibodies has been reported in a group of thalassemia patients which became HIV positive by blood transfusion and in infant AIDS and an ARC group [R. Guroff et al., J. Immunol., 138, 3731 (1987); R. Guroff et al., Pediatric Research, inpress]. In both cases, it has been reported that the clinical symptoms were not serious and rather good in cases where neutralizing antibodies could be detected but got worse in cases where the neutralizing antibodies could not be detected, which suggests that neutralizing antibodies are indeed effective in vivo. There is also a report suggesting that an active immunization should even be done in humans affected with the HIV [Salk, J., Nature, 327, 473 (1987)]. Thus, HIV neutralizing antibodies are important for therapy and prevention of AIDS.

As mentioned above, anti-HIV antibodies are a potential agent for preventing expansion of the disease and for removing affected cells in vivo and they are expected to show an excellent effect when used in combination with the antiviral agent and the like which have already been clinically used.

As the above anti-HIV antibody, monoclonal antibodies having the neutralizing activity against HIV can be used. The fundamental techniques for preparing monoclonal antibodies have already been established, but most of these are concerned with mouse monoclonal antibodies. In application of these mouse monoclonal antibodies In the medical field, i.e. in the immunological diagnosis, the treatment and prevention of diseases, the application of these mouse monoclonal antibodies is regarded as still being unsuitable for practical use. This is due to their physiological activity (e.g. complement-activating activity or ADCC) or antigenicity (mouse monoclonal antibodies induce side-effects such as anaphylactic shock or serum disease when administered to humans as a foreign protein). Human monoclonal antibodies are free from these problems and are expected to be of practical use.

Since multiple injections of the antibodies are necessary in immunotherapy protocols, a reduction in antigenicity is an important concern. With the availability of monoclonal antibodies, many new applications of serum therapy would become well worth exploring if only hypersensitivity reactions could be avoided. Human immunoglobulin is presumably superior to that of other species when administered to humans because it may function better with the recipient's effector cells and be less immunogenic. The approach is to use human monoclonal antibodies, produced either by human hybridomas or by Epstein-Barr virus immortalized human B lymphocyte cell lines. However, at present the techniques for preparing human monoclonal antibodies are not well developed as compared with those for preparing mouse monoclonal antibodies and thus, it is still difficult to obtain human monoclonal antibodies having a higher specificity than that of mouse monoclonal antibodies. Further, there are ethical problems in in vivo immunization and difficulties in in vitro immunization required as the source of human monoclonal antibody producers. That is, in case of the antibodies against a dangerous antigen such as HIV, it is not advisable to use human lymphocytes for producing monoclonal antibodies in viewpoint of biohazards. Therefore, there are only a few HIV neutralizing human monoclonal antibodies which can be used for therapy and prevention.

Chimeric antibodies are considered an approach to solve this problem. As is well known, immunoglobulin molecules consist of variable region and constant region domains. The constant domains of mouse immunoglobulin molecules are probably an important target for the antigenic recognition by the human immune system. If the constant domains of murine antibodies could be replaced by their human counterparts, the resultant chimeric mouse-human molecules would be expected to retain the original specificity but have a much lower antigenicity to humans. Technologies in gene manipulation have now been available which permit the preparation of artificial chimeric mouse-human antibodies [Neuberger et al., Nature 312, 604 (1984); Boulianne et al., Nature, 312, 643 (1984); Morrison et al., Proc. Natl. Acad. Sci. USA, 81, 6851 (1984)].

On the other hand, an analytical research of the immunoglobulin gene has made much progress as development of the genetic engineering techniques in recent years [Susumu Tonegawa, Nature, 307, 575 (1983); Tasuku Honjo, Annual Rev. Immunol. 1, 499 (1983)]. As is well known, immunoglobulins are encoded by genes for the variable region (V region) which binds to the antigen and by genes for the constant region (C region) which has a physiological activity associated with the interaction with a component of the complement system or with a specific cell. Functional immunoglobulin genes are formed by somatic recombination events, which fuse gene segments (V, variable region; D, diversity segment; J, joining region; C, constant region) that are widely separated in the germ-line DNA. Thus, the V-J and V-D-J joinings are essential for the formation of the DNA sequence coding for the variable region of the light (L) chain and the heavy (H) chain, respectively, while switch recombination replaces the coding sequences of the C region of the H chain classes (isotypes). The most important function of the V-J and V-D-J joinings is to create complete immunoglobulin genes with a diverse set of V region-coding DNA sequences from the limited number of gene segments carried in the germ-line genome. Therefore, when an antibody binds specifically to a certain epitope, the V region-coding DNA sequences of the L chain and the H chain, respectively, are almost unique sequences against the epitope.

Each V region gene is formed in such a way that one gene fragment is selected from a number of V gene fragments, D gene fragments (not found for the L chain) and J gene fragments and which are then coupled to each other in this order. In this case, the specificity of an antibody strongly depends on which gene fragment is selected from each group of gene fragments, in other words, the specificity of an antibody is determined by the combination of the gene fragments in the V region gene of the H chain and L chain. Therefore, it is presumed that a particular combination of V-D-J (or V-J) gene fragments of the H chain and the L chain corresponds to a specific antigen.

There exist DNA sequences upstream and downstream of the V region gene, which may play an important role in the expression of the immunoglobulin gene. That is, a DNA sequence exists upstream of the V region gene which acts as a promoter, and downstream of the V region gene (between the J gene fragment and the C region gene) a DNA sequence exists which acts as an enhancer enhancing the transcription efficiency of the immunoglobulin gene. These promotor and enhancer DNA sequences have already been identified and determined in both mouse and human immunoglobulin genes [C. Queen et al., Cell, 33, 741 (1983); J. Banerji et al., Cell, 33, 729 (1983); S. D. Gillies et al., Cell, 33, 717 (1983); A. C. Hayday et al., Nature, 307, 334 (1984); and L. Emorine et al., Nature, 304, 447 (1983)].

In order to solve the above-mentioned problems of both mouse monoclonal antibodies and human monoclonal antibodies, there is an approach to prepare a mouse-human chimeric antibody wherein the variable (V) region for binding to an antigen is derived from a mouse monoclonal antibody but the constant (C) region concerning the antigenicity or immunogenicity and physiological activity is derived from a human monoclonal antibody as described in Japanese Patent First Publications No. 155132/1985 and No. 47500/1986. It should be noted that it is most important for preparing such a chimeric antibody to provide genes (DNA sequences) coding for the amino acid sequences of the variable region of the antibody molecule which is capable of binding to a desired antigen. Up to the present invention, however, it has been very difficult to find such genes (DNA sequences) coding for the amino acid sequences of the variable region of anti-HIV antibodies, and even more difficult of an antibody having a neutralizing activity against HIV. Therefore, there has not yet been any report on a chimeric antibody effective for HIV, much less a preparation of a chimeric anti-HIV antibody having neutralizing activity against HIV.

The present inventors have intensively studied to prepare chimeric antibodies having a neutralizing activity against HIV. As a result, genes (DNA sequences) coding for the variable region of an antibody having a neutralizing activity against HIV was isolated from a hybridoma cell which produces a monoclonal antibody having a neutralizing activity against HIV, and chimeric anti-HIV mouse-human antibodies having neutralizing activity against HIV were prepared by expression of the thus prepared genes.

An object of the present invention is to provide a gene fragment coding for the variable region of the H chain of an immunoglobulin having a neutralizing activity against HIV, which contains DNA sequences coding for the following amino acid sequences (a) to (d):
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser.

Furthermore it is an object of the present invention to provide a gene fragment coding for the variable region of the L chain of an immunoglobulin having a neutralizing activity against HIV, which contains DNA sequences coding for the following amino acid sequences (a′) to (d′):
(a′) Lys Ala Ser Gln Ser Val Asp Tyr Asp Gly Asp Ser Tyr Met Asn;
(b′) Tyr Ala Ala Ser Asn Leu Glu Ser;
(c′) Gln Gln Ser Asn Glu Asp Pro; and (d′) Phe Thr Phe Gly Ser Gly Thr Lys Leu Glu Ile Lys.

Another object is to provide chimeric anti-HIV antibodies which comprise a variable (V) region derived from the antigen-binding site of a mouse monoclonal antibody having an anti-HIV neutralizing activity and a constant (C) region derived from a human monoclonal antibody, wherein the variable regions of the H chain and the L chain contain the following amino acid sequences, respectively:
[Amino acid sequence contained in the variable region of the H chain]
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser;
[Amino acid sequence contained in the variable region of the L chain]
(a′) Lys Ala Ser Gln Ser Val Asp Tyr Asp Gly Asp Ser Tyr Met Asn;
(b′) Tyr Ala Ala Ser Asn Leu Glu Ser;
(c′) Gln Gln Ser Asn Glu Asp Pro;
(d′) Phe Thr Phe Gly Ser Gly Thr Lys Leu Glu Ile Lys.

Still another object of the present invention is to provide a transformed myeloma cell which is cotransformed with: an expression vector which contains a gene coding for the H chain of a chimeric anti-HIV antibody, the gene comprising a gene fragment coding for a human immunoglobulin H chain constant region linked to the 3′-end (downstream) of a gene fragment coding for an H chain variable region of an immunoglobulin having an anti-HIV neutralizing activity, and a promotor upstream of said gene; and an expression vector which contains a gene coding for the L chain of a chimeric anti-HIV antibody, the gene comprising a gene fragment coding for a human immunoglobulin L chain constant region linked to the 3′-end of a gene fragment coding for an L chain variable region of an immunoglobulin having an anti-HIV neutralizing activity, and a promotor upstream of said gene.

A further object of the present invention is to provide a transformed myeloma cell which is transformed with an expression vector which contains a gene coding for a chimeric anti-HIV antibody H chain, the gene comprising a gene fragment coding for a human immunoglobulin H chain constant region linked to the 3′-end of a gene fragment coding for an H chain variable region of an immunoglobulin having an anti-HIV neutralizing activity, and a promotor upstream of said gene coding for the chimeric anti-HIV antibody H chain; and a gene coding for a chimeric anti-HIV chimeric anti-HIV antibody, the gene comprising a gene fragment coding for a human immunoglobulin constant region linked to the 3'-end of a gene fragment coding for an L chain variable region of an immunoglobulin having an anti-HIV neutralizing activity, and a promotor upstream of said gene coding for the L chain of a chimeric anti-HIV antibody.

Still further object of the present invention is to provide a process for preparing a chimeric anti-HIV antibody which comprises simultaneously expressing in a mouse myeloma cell a gene coding for the H chain of a chimeric anti-HIV antibody wherein a gene fragment coding for a human immunoglobulin H chain constant region is linked to the 3'-end (downstream) of a gene fragment coding for an H chain variable region of an immunoglobulin having an anti-HIV neutralizing activity, and a gene coding for the L chain of a chimeric anti-HIV antibody wherein a gene fragment coding for a human immunoglubulin L chain constant region is linked to the 3'-end (downstream) of a gene fragment coding for an L chain variable region of an immunoglobulin having an anti-HIV neutralizing activity, and collecting the expressed product.

Since the chimeric anti-HIV antibodies according to the present invention retain their original specificity, thereby showing an anti-HIV neutralizing activity, but have a much lower antigenicity to humans, they can be used as an agent for diagnosis, treatment and prevention of AIDS which is safe and causes no side effects.

### Brief Description of the Drawings

Figs. 1 and 3 show restriction maps of a VH region gene and a Vκ region gene prepared in the Examples, respectively.

Fig. 2 shows an illustration of an X-ray photograph which denotes the result of a Northern hybridization analysis of a VH region gene prepared in Example 2 with a 54′CBl cell mRNA.

Fig. 4 shows an illustration of an X-ray photograph which denotes the result of a Northern hybridization analysis of a Vκ region gene prepared in Example 2 with a 54′CBl cell mRNA.

Figs. 5 and 7 show one example of a DNA sequence of a VH region gene and a Vκ region gene of the present invention.

Figs. 6 and 8 show one example of an amino acid sequence of a VH region gene and a Vκ region gene of the present invention.

Fig. 9 shows one embodiment of the construction of an expression vector containing a chimeric anti-HIV antibody H chain gene.

Fig. 10 shows one embodiment of the construction of an expression vector containing a chimeric anti-HIV antibody L chain gene.

Fig. 11 shows a further embodiment of the construction of an expression vector containing a chimeric anti-HIV antibody L chain gene.

Fig. 12 shows one embodiment of the construction of an expression vector containing a chimeric anti-HIV antibody H chain gene and a chimeric anti-HIV antibody L chain gene.

Fig. 13 shows an illustration of an X-ray photograph which denotes the result of a Northern hybridization analysis of mRNA of a cell producing a chimeric anti-HIV antibody with a VH region gene, Vκ region gene, both genes being prepared in Example 2, a human Cγl region gene and a Cκ region gene, both genes being prepared in Example 3.

Fig. 14 shows an illustration which denotes the result of a Western blot analysis of a chimeric anti-HIV antibody protein employing an anti-human IgG antibody.

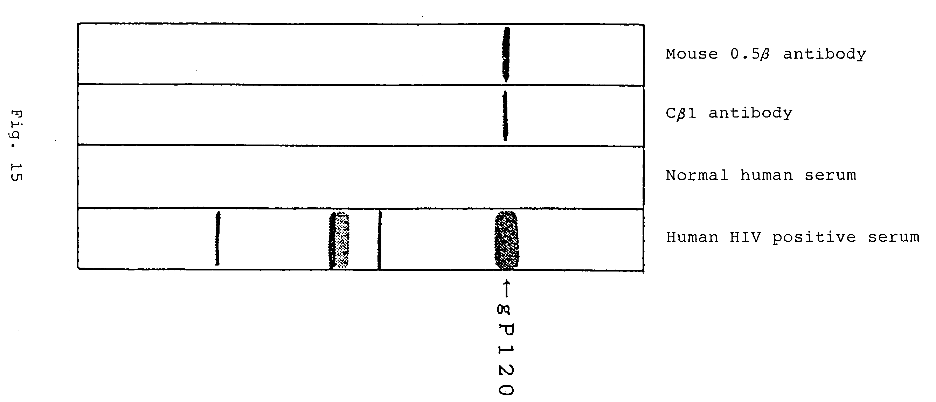

Fig. 15 shows an illustration which denotes the result of a Western blotting analysis of a solubilized HIV particle employing a chimeric anti-HIV antibody.

Fig. 16 shows a graph which denotes the result of a facster analysis of an HIV-affected cell employing a chimeric anti-HIV antibody.

Fig. 17 is a photograph of cells which shows the capability of an anti-HIV antibody to inhibit syncytium formation.

Fig. 18 is a photograph of cells which shows the capability of an anti-HIV antibody to inhibit HIV infection.

An anti-HIV mouse monoclonal antibody-producing cell used in the present invention can be prepared in accordance with the conventional procedures for preparing mouse monoclonal antibodies. For example, an anti-HIV mouse monoclonal antibody-producing cell can be prepared by using the HIV or the HIV glycoprotein (env; gp41, gp120) as an antigen in accordance with the conventional procedures for preparing hybridomas. The HIV or HIV glycoprotein can be prepared from available HIV-affected cells such as H9/HTLV-IIIB (ATCC No. CRL8543) or Molt3/HTLV-IIIB (ATCC No. CRL 8602). The thus prepared anti-HIV mouse monoclonal antibody-producing cells are screened for cells producing a monoclonal antibody having neutralizing activity against HIV. From these cells the hybridoma 54'CBl cell line which produces an antibody having neutralizing activity against HIV had been obtained [Shuzo Matsushita et al., Medical Immunology 14 (1987), p. 307], which is the preferred cell line for the preparation of the gene fragment of the present invention.

The gene fragments coding for the variable region of the present invention are gene fragments separated from such a cell as mentioned above which produces an anti-HIV neutralizing monoclonal antibody and are determined for their nucleotide sequence. As already mentioned, however, since such a cell contains a large number of V region gene fragments in addition to the gene fragments coding for the V region characteristic of the desired anti-HIV antibody (for example, the mouse gene cluster encoding the VH chain of an antibody contains 100 or more V gene fragments, 11 or more D gene fragments and 4 J gene fragments; and the mouse gene cluster encoding the Vκ chain contains 300 or more V gene fragments and 4 J gene fragments), it is necessary to separate the genes coding for the V region characteristic of the desired anti-HIV antibody from other chromosomal genes contained in the cells. There can be employed two processes for isolating the genes coding for the variable (V) region of the H chain and L chain of a mouse immunoglobulin. One method is cloning of the V region genes from the chromosomal DNA of the cell in accordance with the usual procedure [cf. for example, T. Maniatis et al., "Molecular Cloning", Cold Spring Harbor Lab. (1982)]. The other method is to synthesize cDNA from mRNA of the cell in accordance with the usual procedure [for example, D. M. Glover ed. "DNA cloning Vol. I" IRL press (1985)] and to clone the V region genes therefrom. In either one of the methods a DNA probe can be used as a probe for cloning the V region genes which is synthesized based on a nucleic acid sequence of mouse immunoglobulin genes which nucleic acid sequence has already been reported [for example, Sakano et al., Nature, 286, 676 (1980); E. E. Max et al., J. Biol. Chem., 256, 5116 (1981)]. In the former method, an active and expressible V region gene, where the V-D-J genes are rearranged, transcribed to mRNA and translated into proteins, is identified by the Southern hybridization procedure in accordance with the usual method [cf. for example T. Maniatis et al., "Molecular Cloning", Cold Spring Harbor Lab. (9182)] by employing antibody-producing cells and chromosomal DNA of the parent cell to determine the gene characteristic for the antibody-producing cell, enabling faster cloning of the desired V region gene. When the desired gene is prepared from the chromosomal DNA, the produced gene contains intervening sequences called "introns".

The thus cloned gene fragments coding for the V region of the antibody having an anti-HIV neutralizing activity were analyzed by comparing them with other gene fragments coding for antibodies not having an anti-HIV neutralizing activity. As a result, it was found that the gene fragments coding for the V region of the anti-HIV antibody of the present invention are characteristic in that the gene coding for a H chain V region contains a DNA sequence coding for the following amino acid sequence:
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser;
and in that the gene coding for the L chain V region contains a DNA sequence coding for the following amino acid sequence:
(a′) Lys Ala Ser Gln Ser Val Asp Tyr Asp Gly Asp Ser Tyr Met Asn;
(b′) Tyr Ala Ala Ser Asn Leu Glu Ser;
(c′) Gln Gln Ser Asn Glu Asp Pro; and
(d′) Phe Thr Phe Gly Ser Gly Thr Lys Leu Glu Ile Lys.

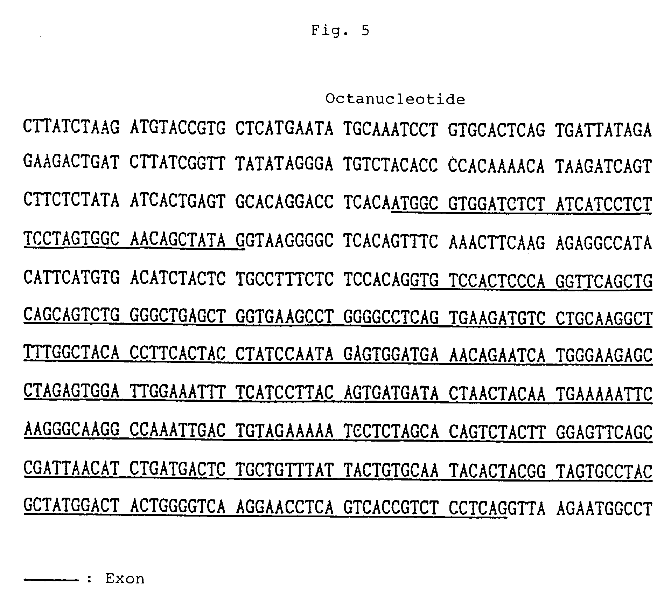

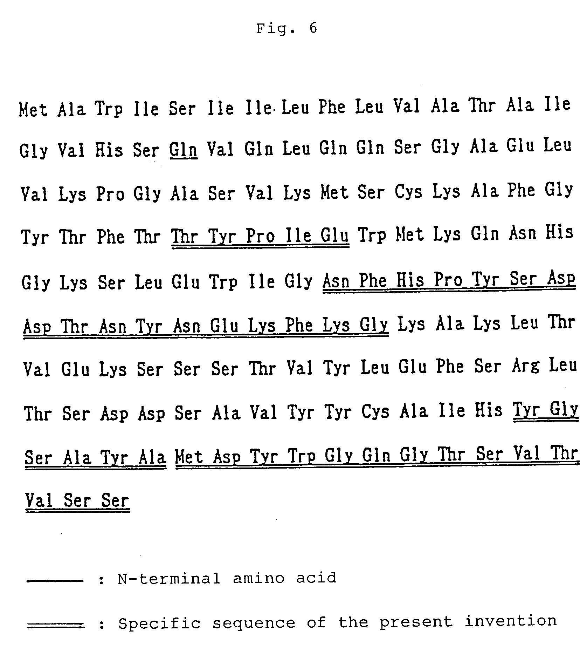

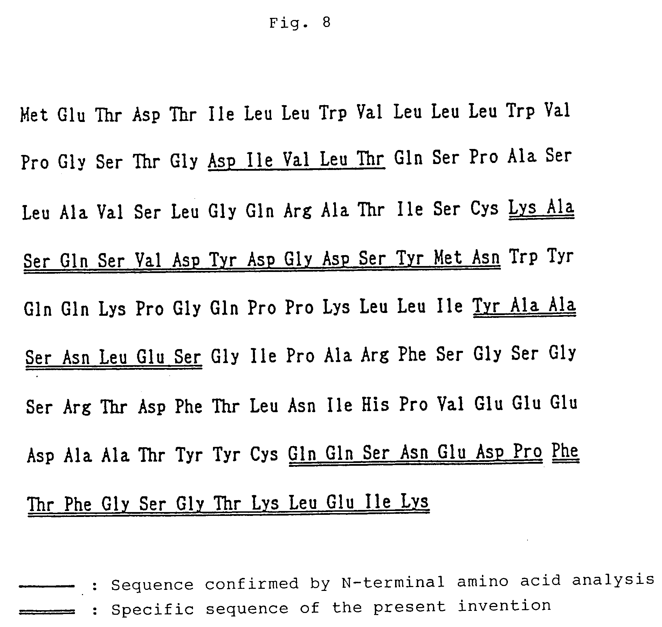

Each of the above four amino acid sequences contained in the H chain and the L chain are considered to be important amino acid sequences for determining the neutralizing activity of the antibody molecule and to be closely related with the function of the antibody molecule having the anti-HIV neutralizing activity. Therefore, the chimeric anti-HIV antibody of the present invention is characterized in that the H chain and the L chain V regions contain DNA sequences coding for the above amino acid sequences. As the genes coding for the V region of the antibody molecule having the anti-HIV neutralizing activity, preferred examples are a gene fragment coding for the amino acid sequence shown in Fig. 6 for the H chain gene and a gene fragment coding for the amino acid sequence shown in Fig. 8 for the L chain gene, respectively. Concerning the nucleotide sequence of such genes, Fig. 5 and Fig. 6 show one example of a nucleotide sequence for the H chain and the L chain gene, respectively.

On the other hand, the constant (C) region genes of the human immunoglobulin H chain gene and L chain gene used for preparing the chimeric anti-HIV antibody of the present invention can be isolated from a human antibody-producing cell such as ARH-77 cell line (ATCC CRL1621) in the same manner as in the case of the V region gene. Human antibody-producing cells are not necessarily employed for isolating the human C region gene since the C region gene is not rearranged. Like the separation of the above-mentioned mouse V region gene, the human C region gene can be isolated by two methods. In either one of the methods a DNA probe can be used which is synthesized based on a nucleic acid sequence of human immunoglobulin gives which nucleic acid sequence has already been reported [for example, J. M. Ellison et al., Nuc. Acids. Res., 10, 4071 (1982); P.A. Heiter et al., Cell, 22, 197 (1980)]. The C region gene is not limited to the γl and κ isotype but also includes genes of the µ, ε, α, γ2-4 and λ isotype. However, the γl chain gene is preferred if a higher complement-activating effect and ADCC are desired.

The chimeric anti-HIV antibody genes can be basically constructed by combining the above-mentioned two gene fragments (V region gene fragment and C region gene fragment) to each other in both cases of the H chain gene and the L chain gene. Furthermore, there are two combinations of these gene fragments depending on the isolating procedure of the gene, i. e. a combination of the V region gene and the C region gene isolated from chromosomal DNA and a combination of the V region gene and the C region gene isolated from cDNA.

When the V region gene isolated from a mouse chromosomal DNA is combined with the C region gene isolated from a human chromosomal DNA, the mouse V region gene preferably contains regulatory elements such as a promoter and an enhancer necessary for expression. Such promoter and enhancer are not necessarily derived from mouse but may be derived from humans or viruses. Preferably, the promoter is situated upstream (5′ side) of the V region gene and the enhancer is situated, but not limited to, between the V region gene and the C region gene.

On the other hand, when the V region gene isolated from mouse cDNA is combined with the C region gene isolated from human cDNA, the combination should be made in such a way that the reading frames of the amino acid sequence encoded by the V region gene and the amino acid sequence encoded by the C region gene are not shifted and that the V region amino acid sequence and the C region amino acid sequence are not changed. Further, it is necessary to incorporate regulatory elements upstream of the genes (promotor and enhancer) for permitting their expression in animal cells.

The thus prepared chimeric antibody gene may preferably be incorporated into an appropriate vector plasmid with a selection marker such as pSV2-gpt [R. C. Mulligan et al., P.N.A.S. USA, 78, 2027 (1981)] or pSV2-neo [P. J. Southern et al., J. Mol. Appl. Genet., 1, 327 (1982)], or a vector plasmid having a part of a viral gene (e.g. papilloma virus) capable of multiplying in a host cell, with the H chain gene and the L chain gene incorporated separately or simultaneously, to construct a chimeric antibody gene plasmid.

For obtaining the chimeric anti-HIV antibody, an animal host cell is transformed with the thus prepared plasmid containing the chimeric antibody gene. Examples of animal host cells are immortalized mouse cells and other animal cells, preferably B lymphocyte cell lines [e.g. plasma cells such as P3X63Ag8·653 (ATCC CRL 1580), P3X63Ag8U·1 (ATCC CRL 1597), P3/NS1/1-Ag4-1 (ATCC CRL18), Sp2/0-Ag12 (ATCC CRL 1581) or hybridomas. Transformation of the host cell with DNA can be effected by any conventional method including the DEAE-dextrane method, the calcium phosphate transfection method, the protoplast fusion method, and the electroporation method [cf. for example, B. D. Hames et al., ed. "Transcription and Translation" IRL Press (1984)]. When the transformation is conducted with a plasmid containing the chimeric antibody gene of both the H chain and L chain, only one selection marker is sufficient, but when the transformation is conducted with separate plasmids containing the two chimeric antibody genes of the H chain and L chain, two selection markers are required. In the latter case, a sequential transformation method is preferred where the transformation is conducted with one plasmid followed by the transformation with the other plasmid.

The thus transformed cell is cultured under appropriate conditions (for example, in RPMI-1640 medium containing 10 % fetal calf serum) to produce and secrete the chimeric anti-HIV antibody in the same manner as a usual hybridoma. The produced chimeric antibody can be purified in the same manner as in the purification of antibodies.

The thus prepared chimeric antibody of the present invention was confirmed to posess anti-HIV neutralizing activity, and hence, the present invention enables to prepare a chimeric anti-HIV antibody which hitherto has never been prepared. The chimeric anti-HIV antibody of the present invention can be used as an effective AIDS-treating agent which hitherto has never been found. Furthermore the characteristic amino acid sequence or DNA sequence of the variable region of the antibody molecule of the present invention having the anti-HIV neutralizing activity were determined. These data permit the preparation of genetic recombination techniques.

### Example 1 (Preparation of an anti-HIV mouse monoclonal antibody)

An anti-HIV mouse monoclonal antibody-producing hybridoma was prepared as follows, i. e. BALB/c mouse was immunized four times with an immunogen consisting of a purified virus (HTLV (human T cell lymphotropic virus) -IIIB) which has been inactivated by heating and a viral glycoprotein fraction purified with a combination of ConA (concanavalin A)-Sepharose (manufactured by Pharmacia) and immunoaffinity sepharose. Thereafter, spleen cells were taken out and fused with X63 mouse myeloma cells by employing polyethylene glycol (manufactured by Sigma Chemical) and the fused cells were cloned (Robert-Guroff et al., J. Exp. Med., 154, 1957 (1981)). The culture supernatants of the hybridoma clones thus obtained were screened for antibodies to the HTLV-IIIB protein by an enzyme-linked immunosorbent assay (ELISA). The positive clones were further assayed by a Western blotting method and an immunofluorescence surface staining of virus producing cells to establish a hybridoma (designated 54'CBl cell) producing an anti-HIV monoclonal antibody (0.5β antibody) [Shuzo Matsushita et al., Medical Immunology, 3, 14 (1987), Matsushita et al., J. Virology, 62, 2107 (1988)].

By a cross immunization procedure, the molecule recognized by the above monoclonal antibody (0.5β antibody) was confirmed to be identical to that recognized by an anti-gp120 antibody which is found in human serum infected with HIV. Thus, the 0.5β antibody was proved to be indentical to the monoclonal antibody recognizing the external envelope glycoprotein gp120 of HTLV-IIIB or lymphadenopathy-associated virus type 1 (LAVl). The 0.5β antibody inhibits syncytium formation between the HIV-infected cells and CD4 positive cells uninfected with HIV. In a virus neutralization test where a mixture of the 0.5β antibody and cell-free HIV virus is inoculated onto H9 cells, the 0.5β antibody has been proved to inhibit the infection at a concentration as low as 100 ng/ml.

For preparation of the V region gene of the anti-HIV chimeric antibody of the present invention as mentioned hereinbelow, the 54′CBl cell producing the anti-HIV mouse monoclonal antibody (0.5β) having such neutralizing activity was employed.

### Example 2 (Isolation of immunoglobulin V gene from the mouse hybridoma producing anti-HIV antibody)

A mouse immunoglobulin H chain variable (VH) region gene was isolated as follows.

Chromosomal DNAs were prepared from 54′CBl cells, X63 cells and BALB/c mouse liver cells in accordance with the method of N. Blin and D. W. Stafford [Nuc. Acids. Res., 3, 2303 (1976)], and each 10 µg of the prepared chromosomal DNA was digested with restriction enzyme EcoRI (manufactured by Takara Shuzo Co. Ltd.,: the reagents used in the following Examples are manufactured by Takara Shuzo Co. Ltd., or Toyobo Co. Ltd., unless otherwise mentioned). The DNA digested with the restriction enzyme was subjected to 0.7 % agarose gel electrophoresis and transferred onto a Nylon membrane filter (Gene Screen Plus, manufactured by NEN Research Product), followed by Southern hybridization with a [³²P]-labelled synthesized DNA probe containing a mouse JH region gene [Sakano et al., Nature, 286, 676 (1980)]. The Southern hybridization was conducted in accordance with the protocol of a manual attached to the Gene Screen Plus filter. Bands detected for the DNA of each cell were compared with each other, and as a result, a band of 3.3 kb was identified as a band characteristic of the EcoRI-digested DNA of the 54′CBl cell when the mouse JH probe was employed. This band is an active gene which contains a functional VH region gene and participates in the expression of specificity of the anti-HIV antibody produced by the 54′CBl cell. The molecular size of this gene was determined calculated by a marker DNA which is prepared by digesting λ phage DNA with the restriction enzyme Hind III.

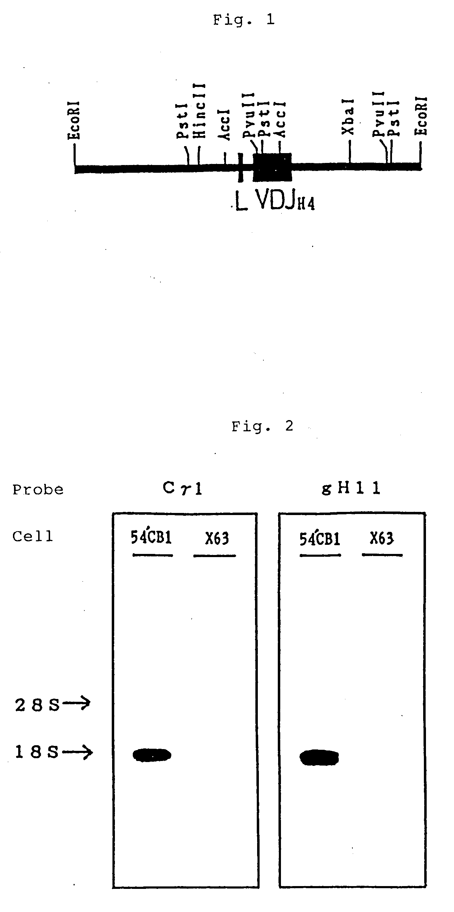

The DNA fragment corresponding to this size was purified by sucrose density gradient centrifugation [sucrose 10 to 40 % (w/v), 26,000 rpm, 18 hours, 15°C]. This DNA fragment and EcoRI arm of λgtll vector DNA (manufactured by Stratagene) were linked to each other with T4 DNA ligase, and an in vitro packaging was carried out with a kit available from Stratagene to prepare a VH gene library of the 54′CBl cell. A plaque hybridization procedure [W. D. Benton and R. W. Davis, Science, 196, 180 (1977)] was conducted on this library and a clone gHll containing the anti-HIV antibody VH region gene was screened by using mouse JH probe. Fig. 1 shows a restriction map of this clone. An EcoRI-inserted fragment of this clone was isolated from phage DNA by the method of Thomas and Davis [M. Thomas and R. W. Davis, J. Mol. Biol., 91, 315 (1974)] and used in the following Northern hybridization.

Total RNA was extracted from the 54′CBl cells, and the X63 cells by the guanidinium isothiocyanate method [J. M. Chirgwin et al., Biochemistry, 18, 5294 (1979)]. The obtained RNA (10 µg) was subjected to electrophoresis with an 0.75 % agarose gel containing 3 % formaldehyde and transferred onto a nylon membrane filter (Gene Screen Plus), followed by Northern hybridization with a [³²P]-labelled synthesized DNA probe containing a mouse Cγl region gene [Honjo et al., Cell, 18, 559 (1979)] or a [³²P]-labelled EcoRI-inserted gene fragment of gHll. The Northern hybridization was conducted in accordance with the protocol of the manual attached to the Gene Screen Plus filter. In the hybridization with both probes, a band was detected at 1.8 kb (Fig. 2). Therefore, the clone gH11 contains a functional VH region gene.

On the other hand, a mouse immunoglobulin κ chain variable (Vκ) region gene was isolated in the following manner.

Chromosomal DNAs were prepared from 54′CBl cells, X63 cells and BALB/c mouse liver cells in accordance with the method of N. Blin and D. W. Stafford. Each 10 µg of the prepared chromosomal DNA was digested with the restriction enzyme HindIII. The DNA digested with the restriction enzyme was subjected to a 0.7 % agarose gel electrophoresis and transferred onto a Nylon membrane filter (Gene Screen Plus), followed by Southern hybridization with [³²P]-labelled synthesized DNA probe containing a mouse Jκ region gene [E. E. Max et al., J. Biol. Chem., 256, 5116 (1981)]. Bands detected for the DNA of each cell were compared with each other, and as a result, a band of 3.6 kb was identified as a band characteristic of the HindIII-digested DNA of the 54′CBl cell when the mouse Jκ probe was employed. This band is an active gene which contains a functional Vκ region gene and participated in the expression of specificity of the anti-HIV antibody produced by the 54′CBl cell. The molecular size of this gene was calculated by a marker DNA which is prepared by digesting λ phage DNA with restriction enzyme Hind III.

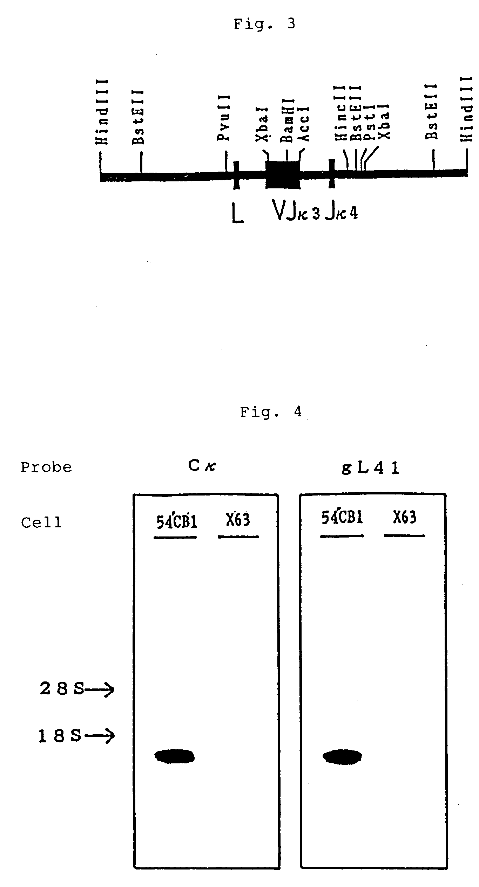

The DNA fragment corrsponding to this size was purified by sucrose density gradient centrifugation [sucrose 10 to 40 % (w/v), 26,000 rpm, 18 hours, 15°C]. This DNA fragment and the HindIII arm of Charon28 vector DNA (manufactured by Bethesda Research Laboratories) were linked to each other with T4 DNA ligase, and an in vitro packaging was carried out with a kit available from Stratagene to prepare a Vκ gene library of the 54′CBl cell. A plaque hybridization procedure by Benton and Davis was conducted on this library and a clone gL41 containing the anti-HIV antibody Vκ region gene was screened by using the mouse Jκ probe. Fig. 3 shows the restriction map of this clone. A HindIII-inserted fragment of this clone was isolated from phage DNA by the method of Thomas and Davis and used in the following Northern hybridization.

Total RNA was extracted from the 54′CBl cells and the X63 cells by the guanidinium isothiocyanate method. The obtained RNA (10 µg) was subjected to an electrophoresis with 0.75 % agarose gel containing 3 % formaldehyde and transferred onto a Nylon membrane filter (Gene Screen Plus), followed by Northern hybridization with a [³²P]-labelled synthesized DNA probe containing the mouse Cκ region gene [E. E. Max et al., J. Biol. Chem., 256, 5116 (1981)] or a [³²P]-labelled HindIII-inserted gene fragment of gL41. In the hybridization with both probes, a band was detected at 1.2 kb (Fig. 4). Therefore, the clone gL41 contains a functional Vκ region gene.

### Example 3 (Isolation of the human immunoglobulin C gene)

The gene containing a human Cγl region-coding gene and the gene containing a human Cκ region-coding gene to be used for the preparation of the anti-HIV chimeric antibody were cloned from human plasma cell leukemia-cell line ARH77 [ATCC CRL 1621] and available from Professor Takeshi Watanabe at Kyushu University, Seitai Bogyo Igaku Kenkyusho (Medical Institute of Bioregulation) [Kudo et al., Gene, 33, 181 (1985); Nishimura et al., Cancer Res., 47, 999 (1987)].

### Example 4 (Determination of the nucleotide sequence of the mouse anti-HIV antibody V region gene)

The nucleotide sequence of the anti-HIV antibody VH region gene was determined in the following manner.

A DNA fragment (HincII-XbaI) of 1.4 kb containing the VH region gene was isolated from the clone gHll. The obtained DNA fragment was blunt-ended at both termini with DNA polymerase large fragment (Takara Shuzo Co. Ltd.,) and recloned into the HincII site of the pUC18 vector to prepare two clones where the DNA fragment was inserted in opposite orientations. These clones were digested with KpnI and BamHI. Deletion mutant clones having a different distance from the BamHI site were prepared with the Takara Kilosequence Deletion Kit and digested with the restriction enzymes EcoRI and HindIII to give VH gene fragments having a different length. These VH gene fragments were inserted into the EcoRI-HindIII site of the M13mp19 vector with the Takara Ligation Kit.

A competent JM101 cell was prepared in accordance with the procedure of the Toyobo instruction manual and transformed with M13mp19 DNA where the VH genes were inserted. A single-stranded DNA was extracted and purified from the transformant and a nucleotide sequence thereof was determined with the Takara M13 Sequencing Kit and the Fuji Gencer Gel System. As a result, the VH gene comprising two exons was confirmed as shown in Fig. 5.

The obtained nucleotide sequence shows the presence of an octanucleotide sequence, ATGCAAAT, believed to be essential to a promoter of immunoglobulin gene, and a VH gene comprising a V gene fragment, a D gene fragment and a JH4 gene fragment (Fig. 5).

Based on this nucleotide sequence, the exons were deduced into an amino acid sequence, and as a result, it is shown that the exons have an open reading frame (Fig. 6). The N-terminal amino acid sequence of the 0.5β antibody H chain was analyzed by the Edman degradation method described in "Zoku Seikagaku Jikken Koza (Biochemistry Experimental Text, Second Volume) No. 2, Chemistry of Proteins, Volume One" ed. by Biochemical Society of Japan, 313 (1987), but could not be determined due to modification of the N-terminal amino acid in some way. The N-terminal amino acid deduced from the nucleotide sequence is glutamine and it was assumed that the susceptibility of this glutamine to be pyroglutamated interrupted the Edman degradation.

On the other hand, a nucleotide sequence of the anti-HIV antibody Vκ region gene was determined in the following manner.

A DNA fragment (BstEII-HincII) of 2.0 kb containing the Vκ region gene was isolated from the clone gL41. The obtained DNA fragment was blunt-ended at both termini with DNA polymerase large fragment (Takara Shuzo Co. Ltd.,) and recloned into the HincII site of the pUC18 vector to prepare two clones where the DNA fragment was inserted in opposite orientations. One (right orientation) of these clones was digested with the restriction enzymes SacI and SmaI and the other clone (opposite orientation) was digested with the restriction enzymes SacI and AccI. Deletion mutant clones having a different distance from the SmaI site or AccI site were prepared with the Takara Kilosequence Deletion Kit and digested with the restriction enzymes EcoRI and HindIII to give Vκ gene fragments having a different length. These Vκ gene fragments were inserted into the EcoRI-HindIII site of the M13mp19 vector with the Takara Ligation Kit.

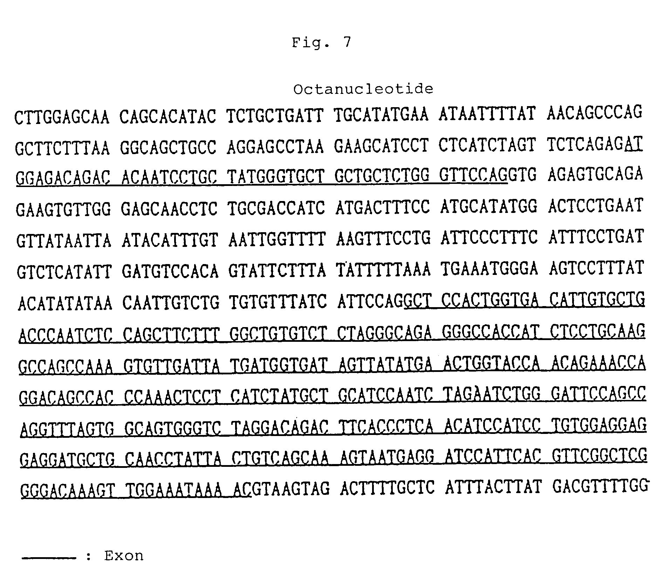

A competent JM101 cell was prepared in accordance with the procedure of the Toyobo instruction manual and transformed with the M13mp19 DNA where the Vκ genes were inserted. A single-stranded DNA was extracted and purified from the transformant and a nucleotide sequence thereof was determined with the Takara M13 Sequencing Kit and the Fuji Gencer Gel System. As a result, the Vκ gene comprising two exons were confirmed as shown in Fig. 7.

The obtained nucleotide sequence shows the presence of the octanucleotide sequence, ATTTGCAT, believed to be essential to a promoter of the immunoglobulin gene, and a Vκ gene comprising a V gene fragment and a Jκ3 gene fragment (Fig. 7).

Based on this nucleotide sequence, the exons were deduced into an amino acid sequence, and as a result, it is shown that the exons have an open reading frame (Fig. 8). The N-terminal amino acid sequence of the 0.5β antibody H chain was analyzed by the Edman degradation method as described above to determine the sequence of five amino acids from the N-terminal (asparagine-isoleucine-valine-leucine-threonine) which agreed with the sequence of the five amino acid deduced from the nucleotide sequence (Fig. 8). It was also confirmed therefrom that this Vκ gene is an expressable gene.

### Example 5 (Construction of plasmid pSV2-βγl containing a gene coding for mouse VH having anti-HIV activity and human Cγl gene)

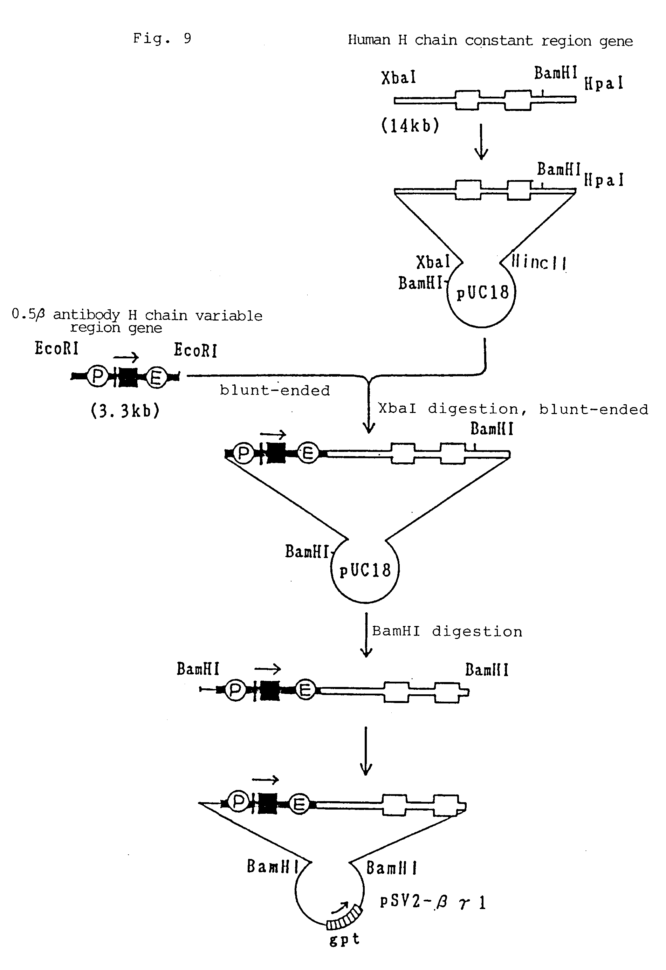

A DNA fragment (XbaI-HpaI) of 14 kb containing the human Cγl gene was inserted into the XbaI-HincII site of the pUC18 vector. The inserted vector was cleaved with the restriction enzyme XbaI and the cleaved site was blunt-ended with T4-DNA polymerase. A DNA fragment (EcoRI-EcoRI) of 3.3 kb containing a mouse H chain promoter region, a mouse VH gene and a mouse H enhancer region was blunt-ended with T4-DNA polymerase at both termini. These two DNA fragments were ligated to each other with the Takara Ligation Kit to prepare a plasmid containing a chimeric antibody H chain gene βγ1. This plasmid was digested with the restriction enzyme BamHI and a βγ1 gene fragment was isolated by means of agarose gel electrophoresis. The obtained fragment was inserted into the BamHI site of the pSV2-gpt vector [R. C. Mulligan et al., P.N.A.S., USA, 78, 2072 (1981)] to prepare pSV2-βγ1 (Fig. 9).

### Example 6 (Construction of plasmids pSV2′-βκ and pSV2-βκ 2 containing a gene coding for mouse Vκ having anti-HIV activity and a human Cκ gene)

pSV2-neo vector [P. J. Southern et al., J. Mol. Appl. Genet., 1, 327 (1982)] containing a EcoRI and BamHI cloning sites was modified to contain an additional HindIII cloning site. Firstly, the pSV2-neo vector was cleaved with the restriction enzyme HindIII and the cleaved site was blunt-ended with T4-DNA polymerase and the blunt-ended site was again ligated to each other with T4-DNA ligase so that the original HindIII site contained in the vector was lost. Then, this vector was digested with the restriction enzymes EcoRI and BamHI and a DNA fragment of about 5.0 kb was isolated by means of agarose gel electrophoresis. This DNA fragment and the EcoRI-BamHI DNA fragment (375 bp) of pBR322 were ligated to each other with the Takara Ligation Kit to construct the pSV2′-neo vector.

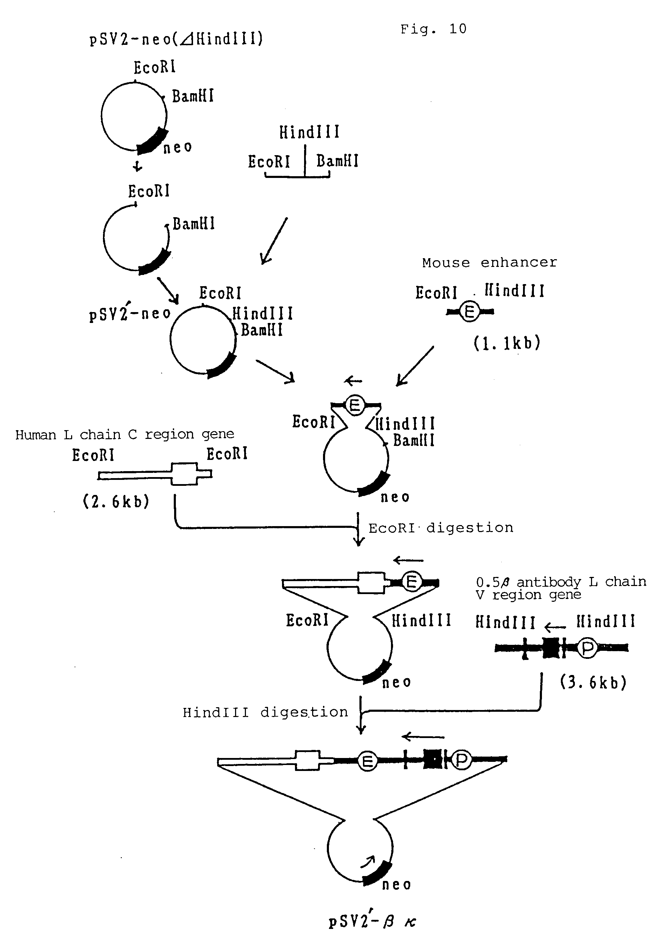

The pSV2′-neo vector was cleaved with the restriction enzymes EcoRI and HindIII. To the cleaved site of the pSV2′-neo vector was ligated a HindIII-EcoRI DNA fragment of 1.1 kb containing a mouse κ chain enhancer region [the original XmnI site has been converted to an EcoRI site with an EcoRI linker; this gene is obtainable from Professor Takeshi Watanabe at Kyushu University, Seitai Bogyo Igaku Kenkyusho (Medical Institute of Bioregulation)] by means of the Takara Ligation Kit. The obtained plasmid was cleaved with the restriction enzyme EcoRI and to the cleaved site was ligated a EcoRI-EcoRI DNA fragment of 2.6 kb containing a human Cκ gene. The resulting plasmid was further cleaved with the restriction enzyme HindIII and to the cleaved site was ligated a HindIII-HindIII DNA fragment of 3.6 kb containing a mouse κ chain promoter region and a mouse Vκ gene to construct pSV2′-βκ (Fig. 10).

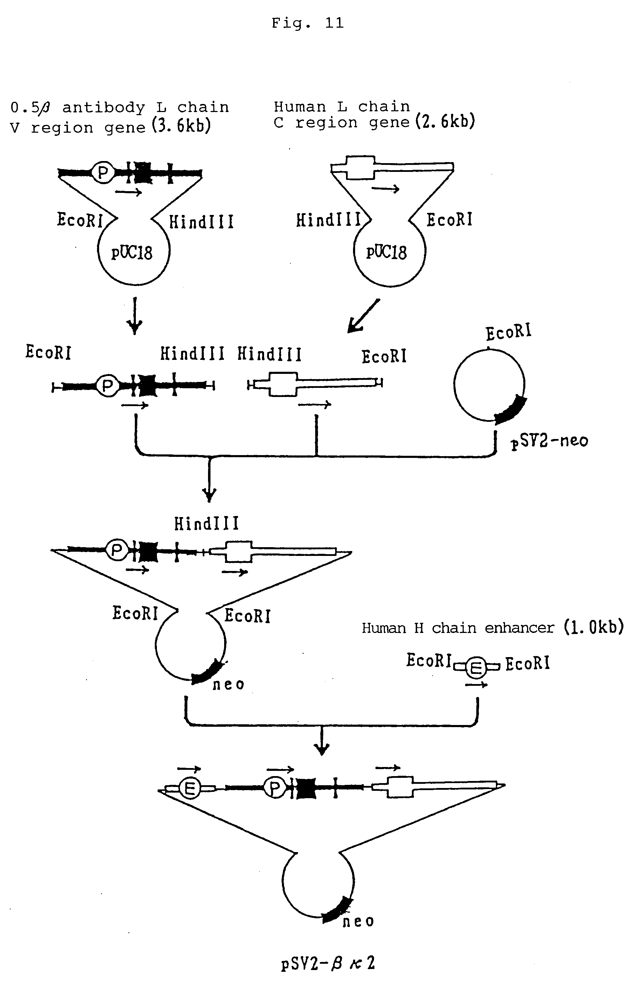

pSV2-βκ2 was also constructed using the human immunoglobulin H chain gene enhancer. A HindIII-HindIII DNA fragment of 3.6 kb containing a mouse κ chain promoter region and a mouse Vκ gene was blunt-ended with T4-DNA polymerase at both termini and inserted into a HincII site of the pUC18 vector to convert the HindIII site at 5′ end of the mouse Vκ gene fragment 5′ end into an EcoRI site. In the same manner, the EcoRI site at 3′ end of an EcoRI-EcoRI DNA fragment of 2.6 kb containing the human Cκ gene was converted into a HindIII site. Both DNA fragments thus prepared were ligated to each other with T4 ligase at the HindIII site and, by utilizing the EcoRI sites at both termini, the ligated DNA fragment was incorporated into the EcoRI site of the pSV2-neo vector. This plasmid was partially digested with the restriction enzyme EcoRI and DNA fragments where only one of the two EcoRI sites was cleaved were isolated by means of agarose gel electrophoresis. To these DNA fragments was incorporated an EcoRI-EcoRI DNA fragment (converted from a MluI-HpaI DNA fragment by utilizing an EcoRI linker) of 1.0 kb containing an enhancer of the human immunoglobulin H chain gene by means of the Takara Ligation Kit. A plasmid where the enhancer DNA fragment was inserted at the 5′ EcoRI site of the mouse Vκ gene was selected to construct pSV2-βκ2 (Fig. 11).

### Example 7 (Construction of plasmid pSV2′-βγ1κ containing a gene coding for mouse-human chimeric H chain having anti-HIV activity and a gene coding for mouse-human chimeric L chain)

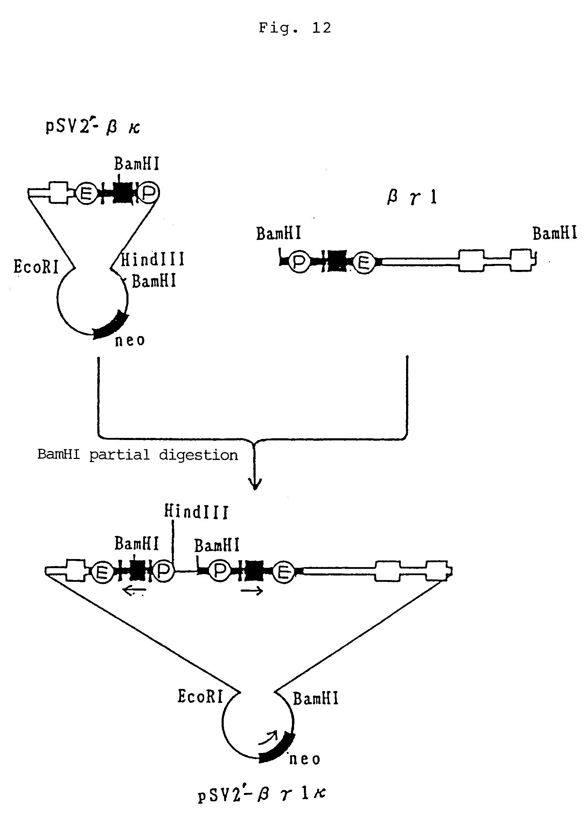

pSV2′-βκ DNA was partially digested with the restriction enzyme BamHI and DNA fragments where only one of the two BamHI sites was cleaved were isolated by means of agarose gel electrophoresis. On the other hand, pSV2-βγ1 DNA was cleaved with the restriction enzyme BamHI and a DNA fragment containing a mouse-human chimeric H chain was isolated by means agarose gel electrophoresis. The thus prepared two DNA fragments were ligated to each other with the Takara Ligation Kit. For screening a plasmid where the DNA fragment containing the mouse-human chimeric H chain gene was inserted at the BamHI site within the pSV2′-neo vector but not at the BamHI site within the mouse Vκ region, the difference of the restriction maps was utilized. In this manner, the plasmid pSV2′-βγ1κ containing both chimeric antibody genes for the H chain and L chain was constructed (Fig. 12).

### Example 8 (Transformation of myeloma with plasmids pSV2-βγ1, pSV2′-βκ and pSV2-βκ2

DNA of pSV2-βγ1, pSV2′-βκ or pSV2-βκ2 was introduced into a variety of myeloma cell lines such as P3X63Ag8.653 (ATCC CRL 1580), P3X63Ag8U·1 (ATCC CRL 1597), P3/NS1/1-Ag4-1 (ATCC CRL 18) and Sp2/0-Ag12 (ATCC CRL 1581) strains [Kohler et al., Nature, 256, 495 (1975); Kohler et al., Eur. J. Immunol., 6, 292 (1976)] by the DEAE-dextran method to carry out transformation. That is, the myeloma cells were washed with DMEM medium not containing fetal calf serum (FCS) for several times and thereafter the cells (1 X 10⁸) were suspended in DMEM 2.5 ml) containing 90 µg of pSV2-βγ1, pSV2′-βκ or pSV2-βκ2 plasmid DNA and 200 µg/ml of DEAE-dextran (Pharmacia) for 30 minutes. After the cells were washed with DMEM not containing FCS for several times, they were suspended in RPMI-1640 medium containing 10 % FCS and transferred to 24-well plate so that each well receives 1 ml containing 5 X 10⁵ cells, followed by culture for 48 hours. Thereafter, the culture medium was replaced with RPMI-1640 medium containing 6.5 µg/ml of mycophenolic acid (Sigma Chemical), 250 µg of xanthin (Sigma Chemical) and 10 % FCS in case that pSV2-βγ1 DNA was introduced, or with RPMI-1640 medium containing 1.5 mg/ml of Geneticin (Sigma Chemical) and 10 % FCS in case that pSV2′-βκ or pSV2-βκ2 DNA was introduced, and the transformed cells were screened.

The transformed cells were fixed on a slide, treated with acetone-ethanol (1:1), and stained with FITC (fluorescein isothiocyanate)-labelled goat anti-human IgG antibody (γ chain specificity or κ chain specificity: Cappel Lab. Inc.) to screen transformant cells having strong and specific fluorescence in cytoplasm, and as a result, establish cells where the introduced DNA is highly expressed to give human γl chain or human κ chain.

pSV2′-βκ or pSV2-βκ DNA was further introduced into the cells transformed with pSV2-βγ1 DNA, and pSV2-βγ1 DNA was further introduced into cells transformed with pSV2′-βκ or pSV2-βκ2 DNA in the same manner as described hereinabove to give sequentially transformed cells.

For screening cells producing a chimeric antibody, an ELISA was carried out with use of anti-human IgG antibody (Cappel Lab. Inc.). That is, the culture supernatant of the sequentially transformed cells was added to a plastic plate (Falcon 3912) coated with anti-human IgG antibody (Cappel Lab. Inc.), reacted with peroxidase-conjugated goat anti-human IgG (Cappel Lab Inc.) and developed with TMBZ (3.3′.5.5′-tetramethylbenzidine: Dojin Kagaku K.K.) to screen and establish chimeric antibody-producing cells.

### Example 9 (Transformation of myeloma with plasmid pSV2′-βγ1κ)

DNA of pSV2′-βγ1κ was introduced into a variety of myeloma cell lines such as P3X63Ag8·653 (ATCC CRL 1580), P3X63Ag8·U1 (ATCC CRL 1597), P3/NS1/1-Ag4-1 (ATCC CRL 18) and Sp2/0-Ag12 (ATCC CRL 1581) strains [Kohler et al., Nature, 256, 495 (1975); Kohler et al., Eur. J. Immunol., 6, 292 (1976)] by DEAE-dextran method to carry out transformation. That is, the myeloma cells were washed with DMEM medium not containing FCS for several times and thereafter the cells (1 X 10⁸) were suspended in DMEM (2.5 ml) containing 90 µg of DNA and 200 µg/ml of DEAE-dextran (Pharmacia) for 30 minutes. After the cells were washed with DMEM not containing FCS for several times, they were suspended in RPMI-1640 medium containing 10 % FCS and transferred to 24-well plate so that each well receives 1 ml containing 5 X 10⁵ cells, followed by culture for 48 hours. Thereafter, the culture medium was replaced with RPMI-1640 medium containing 1.5 mg/ml of Geneticin (Sigma Chemical) and 10 % FCS, and transformed cells were screened.

For screening cells producing a chimeric antibody, an ELISA was carried out with use of anti-human IgG antibody (Cappel Lab. Inc.). That is, to the plastic plate (Falcon 3912) coated with anti-human IgG antibody (Cappel Lab. Inc.) were added the culture supernatant of the transformed cells, peroxidase-conjugated goat anti-human IgG (Cappel lab Inc.) for reaction and TMBZ for development to screen and establish chimeric antibody-producing cells.

### Example 10 (Expression of gene coding for anti-HIV chimeric antibody)

In order to study the expression of the chimeric antibody produced by the thus established chimeric antibody-producing cells, ELISA, Western blotting analysis and Northern blotting analysis were carried out.

After plastic plate (Falcon 3912) was coated with HIV external envelope glycoprotein gp120, anti-human γl chain antibody (Cappel lab. Inc.) or anti-human κ chain antibody (Cappel lab. Inc.), to the plate were added the culture supernatant of the above myeloma transformed with DNA, peroxidase-conjugated goat anti-human IgG (Cappel lab. Inc.) for reaction and TMBZ for development. As a result, the culture supernatant of every chimeric antibody-producing transformant cell reacted with the HIV extenal envelope gp120 glycoprotein, with the anti-human γl chain antibody and the anti-human κ chain antibody. This suggests that the chimeric antibody produced by the chimeric antibody-producing cell has a specificity against gp120, and that the H chain and L chain thereof are similar to those derived from human. Further, the amount of the expressed chimeric antibody was estimated by calibration using human polyclonal IgG, and as a result, it was confirmed that 1 X 10⁷ cells produced the anti-HIV chimeric antibody in 10 ml of culture supernatant in a range of about 0.5 to 5 µg/ml.

From the anti-HIV chimeric antibody-producing cells established in Example 8, cells producing 5 to 8 µg/ml of the chimeric antibody (Cβl cell) were recloned and used in the following experiment. This cell stably produced the chimeric antibody even when cultured in 4ℓ-spiner flask for a month. Such transformant cell Cβl producing anti-HIV neutralizing chimeric antibody has been deposited by the applicant at Fermentation Research Institute, Agency of Industrial Science and Technology, Japan under Budapest Treaty as FERM BP-2249.

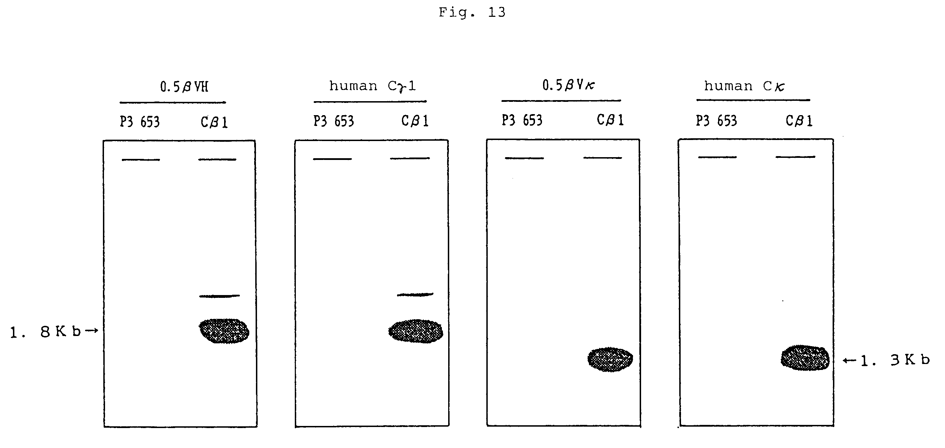

It was studied whether or not mRNA of the chimeric (Cβl) antibody was normally produced. mRNA was extracted from the Cβl antibody-producing cell and its parent cell P3X63Ag8·653 (P3·653). Northern blotting analysis was carried out by using the 0.5β VH gene (gH11), the 0.5β Vκ gene (gL41), the human Cγl chain gene and the human Cκ chain gene as a probe. As a result, a band at about 1.8 kb was detected with the 0.5β VH and human Cκl probes as to H chain while a band at about 1.3 kb was detected with the 0.5β Vκ and human Cκ probe as to L chain, as shown in Fig. 13. This suggested that the chimeric antibody was made chimeric in a mRNA step.

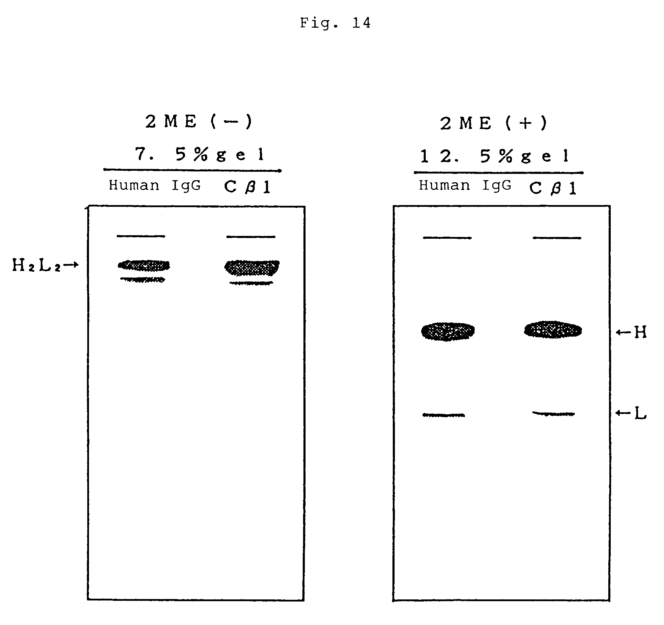

Then, the protein size of the expressed Cβl antibody was studied by using Western blotting analysis. Cβl antibody or normal human IgG antibody was subjected to SDS-PAGE (sodium dodecyl sulfate-polyacrylamide gel electrophoresis analysis in the presence or absence of 2ME and transferred to nitrocellulose filter (Bio Rad). The filter was incubated with goat anti-human IgG (H+L) antibody (Cappel Lab. Inc.) for reaction, peroxidase-conjugated anti-goat IgG antibody (Cappel Lab. Inc.) and HRP Color Development Reagent (Bio Rad) for development. As a result, bands were detected at almost the same position for both Cβl and human IgG, as shown in Fig. 14. The size was about 160 k as H₂L₂, about 50 k as H and about 28 k as L. This suggested that the Cβl chimeric antibody had the normal configuration of an antibody molecule.

### Example 11 (Activity of anti-HIV chimeric antibody)

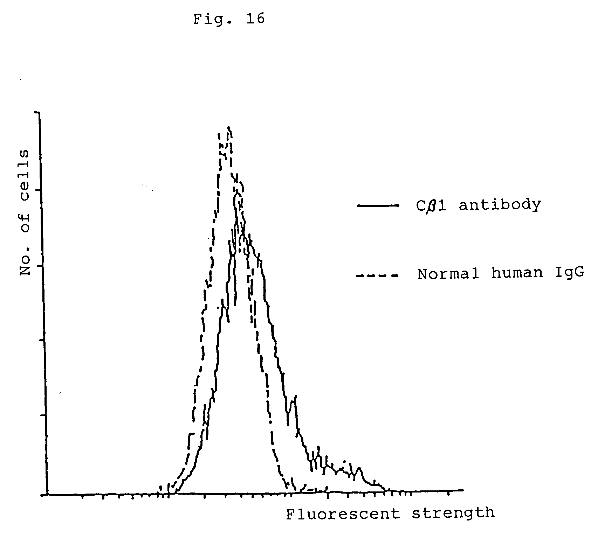

It was studied whether or not the Cβl antibody did react with HIV-infected cell. The Cβl antibody was incubated with HIV-infected H9 cells and analysis was made with a fluorescence activated cell sorter (Facster; Becton Deckinson). As shown in Fig. 16, the Cβl antibody had a shifted fluorescent strength as compared to that of normal human IgG, proving that the Cβl antibody recognized the gp120 antigen on the HIV-infected H9 cells.

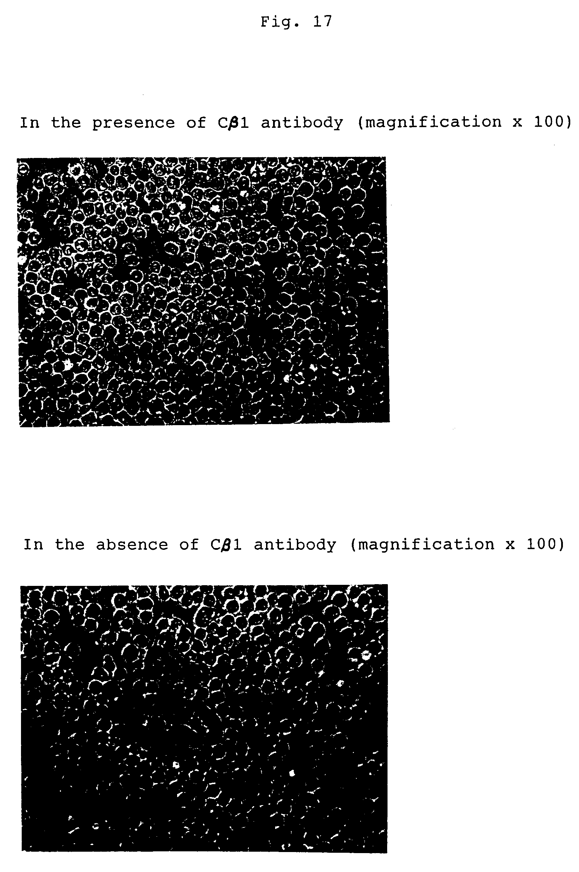

Then, the HIV neutralizing activity of the Cβl antibody was evaluated. Firstly, syncytium formation inhibiting activity of the Cβl antibody was evaluated by using syncytium formation system of HIV-infected cells. That is, when LAVl-infected CEM cells (ATCC CCL119) were previously treated with the Cβl antibody and incubated with uninfected CEM cells to culture together, syncytium formation was inhibited by 100 % as shown in Fig. 17, though the syncytium formation was usually observed 24 hours after LAVl-infected CEM cells were incubated with uninfected CEM cells to culture together. Therefore, the Cβl antibody was expected to have an activity to inhibit a cell-to-cell infection.

The Cβl antibody was then evaluated for its activity to inhibit cell-free HIV particle infection. That is, a giant cell formation was observed as a consequence of the LAVl infection when MT4 cells were infected with HIV (LAVl) particles and cultured for 3 days. A giant cell formation was not observed. In other words, LAV infection did not occur, when LAV was previously treated with the Cβl antibody and incubated with uninfected CEM cells to culture together, syncytium formation was inhibited by 100 % as shown in Fig. 17, though the syncytium formation was usually observed 24 hours after LAVl-infected CEM cells were incubated with uninfected CEM cells to culture together. Therefore, the Cβl antibody was expected to have an activity to inhibit a cell-to-cell infection.

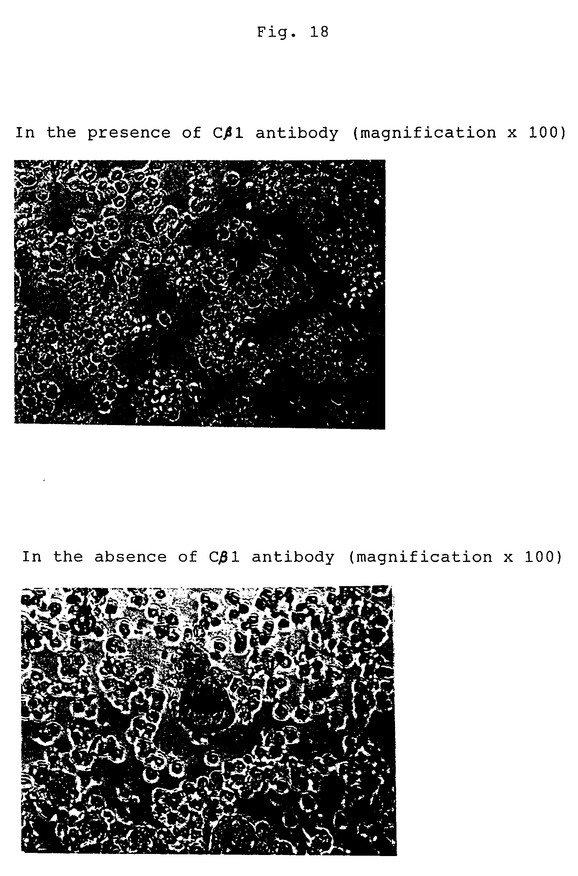

The Cβl antibody was then evaluated for its activity to inhibit cell-free HIV particle infection. That is, a giant cell formation was observed as a consequence of the LAVl infection when MT4 cells were infected with HIV (LAVl) particles and cultured for 3 days. A giant cell formation was not observed. In other words, LAV infection did not occur, when LAV was previously treated with the Cβl antibody (Fig. 18). Furthermore, an indirect flurescent antibody technique using the anti-P24 monoclonal antibody (Cellular Products) was conducted to detect infected cells, but an expression of the P24 antigen was not observed. Consequently, the Cβl antibody appears to have an activity to inhibit HIV particle infection. From the above two results, the Cβl antibody appears to have HIV neutralizing activity.

As clearly shown in the results of Examples 10 and 11, it was proved that the transformant cell of the present invention (Cβl) produced the mouse-human chimeric antibody, comprising the mouse 0.5β antibody variable region (VH, Vκ) capable of specifically binding to the HIV external envelope gp120 glycoprotein and the constant region of the human IgG antibody (Cγl, Cκ), in the form of an intact immunoglobulin (H₂L₂).

## Claims

1. A chimeric anti-HIV antibody which comprises an H chain and an L chain wherein the constant regions of the H chain and the L chain are derived from a human immunoglobulin, and the variable regions of the H chain and the L chain contain the following amino acid sequences:
[Amino acid sequence contained in the H chain variable region]
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser;
[Amino acid sequence contained in the L chain variable region]
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys.

2. The chimeric anti-HIV antibody of claim 1, wherein the H chain and L chain variable regions have the following amino acid sequences:

[H chain variable region]
Gln Val Gln Leu Gln Gln Ser Gly Ala Glu Leu Val Lys
Pro Gly Ala Ser Val Lys Met Ser Cys Lys Ala Phe Gly
Tyr Thr Phe Thr Thr Tyr Pro Ile Glu Trp Met Lys Gln
Asn His Gly Lys Ser Leu Glu Trp Ile Gly Asn Phe His
Pro Tyr Ser Asp Asp Thr Asn Tyr Asn Glu Lys Phe Lys
Gly Lys Ala Lys Leu Thr Val Glu Lys Ser Ser Ser Thr
Val Tyr Leu Glu Phe Ser Arg Leu Thr Ser Asp Asp Ser
Ala Val Tyr Tyr Cys Ala Ile His Tyr Gly Ser Ala Tyr
Ala Met Asp Tyr Trp Gly Gln Gly Thr Ser Val Thr Val
Ser Ser;

[L chain variable region]
Asp Ile Val Leu Thr Gln Ser Pro Ala Ser Leu Ala Val
Ser Leu Gly Gln Arg Ala Thr Ile Ser Cys Lys Ala Ser
Gln Ser Val Asp Tyr Asp Gly Asp Ser Tyr Met Asn Trp
Tyr Gln Gln Lys Pro Gly Gln Pro Pro Lys Leu Leu Ile
Tyr Ala Ala Ser Asn Leu Glu Ser Gly Ile Pro Ala Arg
Phe Ser Gly Ser Gly Ser Arg Thr Asp Phe Thr Leu Asn
Ile His Pro Val Glu Glu Glu Asp Ala Ala Thr Tyr Tyr
Cys Gln Gln Ser Asn Glu Asp Pro Phe Thr Phe Gly Ser
Gly Thr Lys Leu Glu Ile Lys.

3. A transformed myeloma cell which is cotransformed with: an expression vector which contains a gene coding for the H chain of a chimeric anti-HIV antibody, the gene comprising a gene fragment coding for a human immunoglobulin H chain constant region linked to the 3'-end (downstream) of a gene fragment coding for an H chain variable region of an immunoglubulin having an anti-HIV neutralizing activity, which contains DNA sequences coding for the following amino acid sequences (a) to (d):
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser , and a promotor
upstream of said gene; and an expression vector which contains a gene coding for the L chain of a chimeric anti-HIV antibody, the gene comprising a gene fragment coding for a human immunoglobulin L chain constant region linked to the 3'-end of a gene fragment coding for an L chain variable region of an immunoglubulin having an anti-HIV neutralizing activity, which contains DNA sequences coding for the following amino acid sequences (a') to (d'):
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys,
and a promotor upstream of said gene.

4. A transformed myeloma cell which is transformed with an expression vector which contains a gene coding for the H chain of a chimeric anti-HIV antibody, the gene comprising a gene fragment coding for a human immunoglobulin H chain constant region linked to the 3'-end of a gene fragment coding for an H chain variable region of an immunoglubulin having an anti-HIV neutralizing activity, which contains DNA sequences coding for the following amino acid sequences (a) to (d):
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser , and a promotor upstream of said gene
coding for the H chain of the chimeric anti-HIV antibody, and a gene coding for the L chain of a chimeric anti-HIV antibody, the gene comprising a gene fragment coding for a human immunoglobulin L chain constant region linked to the 3'-end of a gene fragment coding for an L chain variable region of an immunoglubulin having an anti-HIV neutralizing activity, which contains DNA sequences coding for the following amino acid sequences (a') to (d'):
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys, and a promotor
upstream of said gene coding for the L chain of the chimeric anti-HIV antibody.

5. A process for preparing a chimeric anti-HIV antibody which comprises simultaneously expressing in a mouse myeloma cell a gene coding for the H chain of a chimeric anti-HIV antibody wherein a gene fragment coding for a human immunoglobulin H chain constant region is linked to the 3'-end (downstream) of the gene fragment coding for an H chain variable region of an immunoglubulin having an anti-HIV neutralizing activity, which contains DNA sequences coding for the following amino acid sequences (a) to (d):
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser,
and a gene coding for the L-chain of a chimeric anti-HIV antibody wherein a gene fragment coding for a human immunoglobulin L chain constant region is linked to the 3'-end of the gene fragment coding for an L chain variable region of an immunoglubulin having an anti-HIV neutralizing activity, which contains DNA sequences coding for the following amino acid sequences (a') to (d'):
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys, secreting and collecting the expressed product.

## Patentansprüche

1. Chimerer anti-HIV-Antikörper, umfassend eine H-Kette und eine L-Kette, wobei die konstanten Regionen der H-Kette und der L-Kette von einem menschlichen Immunglobulin stammen und die variablen Regionen der H-Kette und der L-Kette die folgenden Aminosäuresequenzen enthalten:
[Aminosäuresequenz in der variablen Region der H-Kette]
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser;
[Aminosäuresequenz in der variablen Region der L-Kette]
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys.

2. Chimerer anti-HIV-Antikörper nach Anspruch 1, wobei die variablen Regionen der H-Kette und der L-Kette die folgenden Aminosäuresequenzen aufweisen:

[variable Region der H-Kette]
Gln Val Gln Leu Gln Gln Ser Gly Ala Glu Leu Val Lys
Pro Gly Ala Ser Val Lys Met Ser Cys Lys Ala Phe Gly
Tyr Thr Phe Thr Thr Tyr Pro Ile Glu Trp Met Lys Gln
Asn His Gly Lys Ser Leu Glu Trp Ile Gly Asn Phe His
Pro Tyr Ser Asp Asp Thr Asn Tyr Asn Glu Lys Phe Lys
Gly Lys Ala Lys Leu Thr Val Glu Lys Ser Ser Ser Thr
Val Tyr Leu Glu Phe Ser Arg Leu Thr Ser Asp Asp Ser
Ala Val Tyr Tyr Cys Ala Ile His Tyr Gly Ser Ala Tyr
Ala Met Asp Tyr Trp Gly Gln Gly Thr Ser Val Thr Val
Ser Ser;
[variable Region der L-Kette]
Asp Ile Val Leu Thr Gln Ser Pro Ala Ser Leu Ala Val
Ser Leu Gly Gln Arg Ala Thr Ile Ser Cys Lys Ala Ser
Gln Ser Val Asp Tyr Asp Gly Asp Ser Tyr Met Asn Trp
Tyr Gln Gln Lys Pro Gly Gln Pro Pro Lys Leu Leu Ile
Tyr Ala Ala Ser Asn Leu Glu Ser Gly Ile Pro Ala Arg
Phe Ser Gly Ser Gly Ser Arg Thr Asp Phe Thr Leu Asn
Ile His Pro Val Glu Glu Glu Asp Ala Ala Thr Tyr Tyr
Cys Gln Gln Ser Asn Glu Asp Pro Phe Thr Phe Gly Ser
Gly Thr Lys Leu Glu Ile Lys.

3. Transformierte Myelomzelle, die cotransformiert ist mit einem Expressionsvektor, der ein die H-Kette eines chimeren anti-HIV-Antikörpers codierendes Gen enthält, wobei das Gen ein Genfragment umfaßt, das die konstante Region der H-Kette eines menschlichen Immunglobulins codiert, verknüpft mit dem 3'-Ende (stromabwärts) eines Genfragments, das die variable Region einer H-Kette eines Immunglobulins mit anti-HIV-neutralisierender Aktivität codiert, enthaltend DNA-Sequenzen, die die folgenden Aminosäuresequenzen (a) bis (d) codieren:
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser ,
und einen Promotor stromaufwärts dieses Gens; und einem Expressionsvektor, der ein die L-Kette eines chimeren anti-HIV-Antikörpers codierendes Gen enthält, wobei das Gen ein Genfragment umfaßt, das die konstante Region der L-Kette eines menschlichen Immunglobulins codiert, verknüpft mit dem 3'-Ende eines Genfragments, das die variable Region einer L-Kette eines Immunglobulins mit anti-HIV-neutralisierender Aktivität codiert, enthaltend DNA-Sequenzen, die die folgenden Aminosäuresequenzen (a') bis (d') codieren:
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys,
und einen Promotor stromaufwärts dieses Gens.

4. Transformierte Myelomzelle, die mit einem Expressionsvektor transformiert ist, welcher ein die H-Kette eines chimeren anti-HIV-Antikörpers codierendes Gen enthält, wobei das Gen ein Genfragment umfaßt, das die konstante Region einer H-Kette eines menschlichen Immunglobulins codiert, verknüpft mit dem 3'-Ende eines Genfragments, das die variable Region einer H-Kette eines Immunglobulins mit anti-HIV-neutralisierender Aktivität codiert, enthaltend DNA-Sequenzen, die die folgenden Aminosäuresequenzen (a) bis (d) codieren:
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser ,
und einen Promotor stromaufwärts des die H-Kette des chimeren anti-HIV-Antikörper codierenden Gens, und ein die L-Kette eines chimeren anti-HIV-Antikörper codierenden Gens, wobei das Gen ein Genfragment umfaßt, das die konstante Region der L-Kette eines menschlichen Immunglobulins codiert, verknüpft mit dem 3'-Ende eines Genfragments, das die variable Region einer L-Kette eines Immunglobulins mit anti-HIV-neutralisierender Aktivität codiert, enthaltend DNA-Sequenzen, die die folgenden Aminosäuresequenzen (a') bis (d') codieren:
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Phe Thr Phe Gly Ser Gly Thr Lys Leu Glu Ile Lys,
und einen Promotor stromaufwärts des Gens, das die L-Kette des chimeren anti-HIV-Antikörpers codiert.

5. Verfahren zur Herstellung eines chimeren anti-HIV-Antikörpers, umfassend die gleichzeitige Expression in einer Maus-Myelomzelle eines Gens, das die H-Kette eines chimeren anti-HIV-Antikörpers codiert, wobei ein die konstante Region der H-Kette eines menschlichen Immunglobulins codierendes Genfragment mit dem 3'-Ende (stromabwärts) des Genfragments verknüpft ist, das die variable Region der H-Kette eines Immunglobulins mit anti-HIV-neutralisierender Aktivität codiert, enthaltend DNA-Sequenzen, die die folgenden Aminosäuresequenzen (a) bis (d) codieren:
(a) Thr Tyr Pro Ile Glu;
(b) Asn Phe His Pro Tyr Ser Asp Asp Thr Asn Tyr Asn Glu Lys Phe Lys Gly;
(c) Tyr Gly Ser Ala Tyr Ala; and
(d) Met Asp Tyr Trp Gly Gln Gly Thr Ser Val Thr Val Ser Ser,
und ein die L-Kette eines chimeren anti-HIV-Antikörper codierendes Gen, wobei ein Genfragment, das die konstante Region einer L-Kette eines menschlichen Immunglobulins codiert, mit dem 3'-Ende des Genfragments verknüpft ist, das die variable Region einer L-Kette eines Immunglobulins mit anti-HIV-neutralisierender Aktivität codiert, enthaltend DNA-Sequenzen, die die folgenden Aminosäuresequenzen (a') bis (d') codieren:
(a') Lys Ala Ser Gln Ser Val Asp Tyr Asp Gly Asp Ser Tyr Met Asn;
(b') Tyr Ala Ala Ser Asn Leu Glu Ser;
(c') Gln Gln Ser Asn Glu Asp Pro; and
(d') Pho Thr Phe Gly Ser Gly Thr Lys Leu Glu Ile Lys
Sekretion und Sammeln des exprimierten Produkts.

## Revendications

1. Un anticorps anti-HIV chimérique qui comprend une chaîne H et une chaîne L, selon lequel les régions constantes de la chaîne H et de la chaîne L sont dérivées d'une immunoglobuline humaine et les régions variables de la chaîne H et de la chaîne L contiennent les séquences d'aminoacides suivantes :
[Séquence d'aminoacides contenue dans la région variable de la chaîne H]
(a) Thr Tyr Pro Ile Glu ;
(b) Asn Phe His Pro Tyr Ser Asp Asp Thr Asn Tyr Asn Glu Lys Phe Lys Gly ;
(c) Tyr Gly Ser Ala Tyr Ala ; et
(d) Met Asp Tyr Trp Gly Gln Gly Thr Ser Val Thr Val Ser Ser ;
[Séquence d'aminoacides contenue dans la région variable de la chaîne L]
(a') Lys Ala Ser Gln Ser Val Asp Tyr Asp Gly Asp Ser Tyr Met Asn ;
(b') Tyr Ala Ala Ser Asn Leu Glu Ser ;
(c') Gln Gln Ser Asn Glu Asp Pro ; et
(d') Phe Thr Phe Gly Ser Gly Thr Lys Leu Glu Ile Lys.

2. L'anticorps anti-HIV chimérique selon la revendication 1, selon laquelle les régions variables de la chaîne H et de la chaîne L ont les séquences d'aminoacides suivantes :

[Région variable de la chaîne H]
Gln Val Gln Leu Gln Gln Ser Gly Ala Glu Leu Val Lys
Pro Gly Ala Ser Val Lys Met Ser Cys Lys Ala Phe Gly
Tyr Thr Phe Thr Thr Tyr Pro Ile Glu Trp Met Lys Gln
Asn His Gly Lys Ser Leu Glu Trp Ile Gly Asn Phe His
Pro Tyr Ser Asp Asp Thr Asn Tyr Asn Glu Lys Phe Lys
Gly Lys Ala Lys Leu Thr Val Glu Lys Ser Ser Ser Thr
Val Tyr Leu Glu Phe Ser Arg Leu Thr Ser Asp Asp Ser
Ala Val Tyr Tyr Cys Ala Ile His Tyr Gly Ser Ala Tyr
Ala Met Asp Tyr Trp Gly Gln Gly Thr Ser Val Thr Val
Ser Ser ;

[Région variable de la chaîne L]
Asp Ile Val Leu Thr Gln Ser Pro Ala Ser Leu Ala Val
Ser Leu Gly Gln Arg Ala Thr Ile Ser Cys Lys Ala Ser
Gln Ser Val Asp Tyr Asp Gly Asp Ser Tyr Met Asn Trp
Tyr Gln Gln Lys Pro Gly Gln Pro Pro Lys Leu Leu Ile
Tyr Ala Ala Ser Asn Leu Glu Ser Gly Ile Pro Ala Arg
Phe Ser Gly Ser Gly Ser Arg Thr Asp Phe Thr Leu Asn
Ile His Pro Val Glu Glu Glu Asp Ala Ala Thr Tyr Tyr
Cys Gln Gln Ser Asn Glu Asp Pro Phe Thr Phe Gly Ser
Gly Thr Lys Leu Glu Ile Lys.

3. Une cellule de myélome transformée qui est cotransformée avec : un vecteur d'expression qui contient un gène codant pour la chaîne H d'un anticorps anti-HIV chimérique, le gène comprenant un fragment de gène codant pour une région constante de la chaîne H d'une immunoglobuline humaine lié à l'extrémité 3' (en aval) d'un fragment de gène codant pour une région variable de la chaîne H d'une immunoglobuline ayant une activité neutralisante anti-HIV, qui contient des séquences de ADN codant pour les séquences d'aminoacides suivantes (a) à (d) :
(a) Thr Tyr Pro Ile Glu ;
(b) Asn Phe His Pro Tyr Ser Asp Asp Thr Asn Tyr Asn Glu Lys Phe Lys Gly ;
(c) Tyr Gly Ser Ala Tyr Ala ; et
(d) Met Asp Tyr Trp Gly Gln Gly Thr Ser Val Thr Val Ser Ser,
et un promoteur en amont dudit gène; et un vecteur d'expression qui contient un gène codant pour la chaîne L d'un anticorps anti-HIV chimérique, le gène comprenant un fragment de gène codant pour une région constante de la chaîne L d'une immunoglobuline humaine lié à l'extrémité 3' d'un fragment de gène codant pour une région variable de la chaîne L d'une immunoglobuline ayant une activité neutralisante anti-HIV, qui contient des séquences de ADN codant pour les séquences d'aminoacides suivantes : (a') à (d') :
(a') Lys Ala Ser Gln Ser Val Asp Tyr Asp Gly Asp Ser Tyr Met Asn ;
(b') Tyr Ala Ala Ser Asn Leu Glu Ser ;
(c') Gln Gln Ser Asn Glu Asp Pro ; et
(d') Phe Thr Phe Gly Ser Gly Thr Lys Leu Glu Ile Lys,
et un promoteur en amont dudit gène.

4. Une cellule de myélome transformée qui est transformée par un vecteur d'expression qui contient un gène codant pourla chaîne H d'un anticorps anti-HIV chimérique, le gène comprenant un fragment de gène codant pour une région constante de la chaîne H d'une immunoglobuline humaine lié à l'extrémité 3' d'un fragment de gène codant pour une région variable de la chaîne H d'une immunoglobuline ayant une activité neutralisante anti-HIV, qui contient des séquences d'ADN codant pour les séquences d'aminoacides suivantes (a) à (d) :
(a) Thr Tyr Pro Ile Glu ;
(b) Asn Phe His Pro Tyr Ser Asp Asp Thr Asn Tyr Asn Glu Lys Phe Lys Gly ;
(c) Tyr Gly Ser Ala Tyr Ala ; et
(d) Met Asp Tyr Trp Gly Gln Gly Thr Ser Val Thr Val Ser Ser,
et un promoteur en amont dudit gène codant pour la chaîne H de l'anticorps anti-HIV chimérique, et un gène codant pour la chaîne L d'un anticorps anti-HIV chimérique, le gène comprenant un fragment de gène codant pour une région constante de la chaîne L d'une immunoglobuline humaine lié à l'extrémité 3' d'un fragment de gène codant pour une région variable de la chaîne L d'une immunoglobuline ayant une activité neutralisante anti-HIV, qui contient des séquences d'ADN codant pour les séquences d'aminoacides suivantes (a') à (d') :
(a') Lys Ala Ser Gln Ser Val Asp Tyr Asp Gly Asp Ser Tyr Met Asn ;
(b') Tyr Ala Ala Ser Asn Leu Glu Ser ;
(c') Gln Gln Ser Asn Glu Asp Pro ; et
(d') Phe Thr Phe Gly Ser Gly Thr Lys Leu Glu Ile Lys,
et un promoteur en amont dudit gène codant pour la chaîne L de l'anticorps anti-HIV chimérique.

5. Un procédé de préparation d'un anticorps anti-HIV chimérique qui consiste à exprimer simultanément dans une cellule de myélome de souris un gène codant pour la chaîne H d'un anticorps anti-HIV chimérique, selon lequel, un fragment de gène codant pour une région constante de la chaîne H d'une immunoglobuline humaine est relié à l'extrémité 3' (en aval) du fragment de gène codant pour une région variable de la chaîne H d'une immunoglobuline ayant une activité neutralisante anti-HIV, qui contient des séquences d'ADN codant pour les séquences d'aminoacides suivantes (a) à (d) :
(a) Thr Tyr Pro Ile Glu ;
(b) Asn Phe His Pro Tyr Ser Asp Asp Thr Asn Tyr Asn Glu Lys Phe Lys Gly ;
(c) Tyr Gly Ser Ala Tyr Ala ; et
(d) Met Asp Tyr Trp Gly Gln Gly Thr Ser Val Thr Val Ser Ser,
et un gène codant pour la chaîne L d'un anticorps anti-HIV chimérique dans lequel un fragment de gène codant pour une région constante de la chaîne L d'une immunoglobuline humaine est relié à l'extrémité 3' du fragment de gène codant pour une région variable de la chaîne L d'une immunoglobuline ayant une activité neutralisante anti-HIV, qui contient des séquences d'ADN codant pour les séquences d'aminoacides suivantes (a') à (d') :
(a') Lys Ala Ser Gln Ser Val Asp Tyr Asp Gly Asp Ser Tyr Met Asn ;
(b') Tyr Ala Ala Ser Asn Leu Glu Ser ;
(c') Gln Gln Ser Asn Glu Asp Pro ; et
(d') Phe Thr Phe Gly Ser Gly Thr Lys Leu Glu Ile Lys,
à secréter et à collecter le produit exprimé.
